(19) Europäisches Patentamt
European Patent Office
Office européen des brevets

(11) EP 3 420 893 A1

(12) EUROPEAN PATENT APPLICATION

(43) Date of publication:
02.01.2019 Bulletin 2019/01

(51) Int Cl.:
A61B 5/021 (2006.01)  A61B 5/02 (2006.01)
A61B 5/00 (2006.01)  A61B 5/024 (2006.01)
A61B 5/0285 (2006.01)  A61B 5/0295 (2006.01)
A61B 5/11 (2006.01)

(21) Application number: 17179202.1

(22) Date of filing: 30.06.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Nokia Technologies Oy
02610 Espoo (FI)

(72) Inventors:
• Salo, Antti
  08500 Lohja as (FI)
• Rajala, Satu
  02680 Espoo (FI)
• Blomqvist, Kim
  26250 Espoo (FI)

(74) Representative: Swindell & Pearson Limited
48 Friar Gate
Derby DE1 1GY (GB)

(54) METHODS, APPARATUS, COMPUTER PROGRAMS, SYSTEMS FOR CALCULATING A PULSE WAVE VELOCITY OF A SUBJECT

(57) A method comprising: determining a time difference between detecting a heart-related signal of a subject and detecting a vasculature signal of the subject at a first one of a plurality of distributed sensors; using a controller to automatically estimate an in-vivo distance from the heart of the subject to the first one of the plurality of sensors in dependence upon a determined unconstrained body position of the subject; and calculating a pulse wave velocity.

FIG. 1

EP 3 420 893 A1

**Description**

TECHNOLOGICAL FIELD

Embodiments of the present invention relate to methods, apparatuses, computer programs and systems for calculating a pulse wave velocity of a subject. In particular, they relate to calculating the pulse wave velocity of the subject in an unobtrusive manner.

BACKGROUND

In the human circulation system, the heart pumps blood around the vasculature (arteries and veins) of a subject.

The heart pumps rhythmically, intermittently pumping a volume of blood into the arteries of the vasculature. The arteries are elastic tubes and as the volume of blood is passed along the branching network of arteries, the arteries distend increasing their volume. This increase in volume is referred to as an arterial pulse or simply a pulse. The speed at which this pulse moves along the arterial system, the pulse wave velocity, depends in part upon the systolic pressure generated by a ventricular contraction of the heart and the elastic properties of the arteries.

The pulse wave velocity is therefore a clinical indication related to the health of a subject's circulation system. It is dependent upon the systolic blood pressure generated by ventricular contraction and also the health of the vasculature.

The pulse wave velocity is determined by dividing the in-vivo distance from the heart to a particular pulse point of the vasculature of the subject by the time difference between detecting the heart's ventricular output and detecting the arterial pulse at the pulse point. The ventricular output may, for example, be detected by attaching electrocardiogram electrodes to the subject and the arterial pulse may be detected by detecting the change in arterial volume produced by the passing arterial pulse wave (plethysmography). The distance between the heart and the particular pulse point for the subject may, for example, be determined from a look-up table based upon the subject's height. It will be appreciated from the foregoing that it is important that a standard pulse point is used for the measurement of the arterial pulse signal so that the distance used is correct. It is therefore necessary to constrain the relationship between the subject and the sensors used. Typically the sensors are attached to the body of the subject at specific positions.

It would be desirable to be able to measure pulse wave velocity of a subject differently.

BRIEF SUMMARY

According to various, but not necessarily all, embodiments of the invention there is provided a method comprising:

determining a time difference between detecting a heart-related signal of a subject and detecting a vasculature signal of the subject at a first one of a plurality of distributed sensors;

using a controller to automatically estimate an in-vivo distance from the heart of the subject to the first one of the plurality of sensors in dependence upon a determined unconstrained body position of the subject; and

calculating a pulse wave velocity.

According to various, but not necessarily all, embodiments of the invention there is provided a computer program that when run by a processor enables the processor to estimate an in-vivo distance, from a heart to a first one of a plurality of sensors in dependence upon a determined unconstrained body position of the subject, wherein the distance causes a time difference, dependent upon a pulse wave velocity, between detecting a heart-related signal and detecting a vascular signal at the first one of a plurality of distributed sensors.

According to various, but not necessarily all, embodiments of the invention there is provided a system comprising: means for detecting a heart-related signal; means for detecting a vasculature signal at a first one of a plurality of distributed sensors; means for determining a time difference between detecting the heart-related signal and detecting the vasculature signal; means for automatically estimating an in-vivo distance from the heart of the subject to the first one of the plurality of sensors in dependence upon a determined unconstrained body position of the subject; and means for calculating a pulse wave velocity using the determined time difference and the estimated in-vivo distance.

According to various, but not necessarily all, embodiments of the invention there is provided an apparatus comprising:

at least one processor; and
at least one memory including computer program code
the at least one memory and the computer program code configured to, with the at least one processor, cause the apparatus at least to perform:

estimating an in-vivo distance, from a heart to a first one of a plurality of sensors in dependence upon a determined unconstrained body position of the subject, wherein the distance causes a time difference, dependent upon a pulse wave velocity, between detecting a heart-related signal and detecting a vascular signal at the first one of a plurality of distributed sensors.

According to various, but not necessarily all, embodiments of the invention there is provided a system comprising:

one or more sensors configured to detect a heart-related signal;

a plurality of distributed sensors each configured to detect a vasculature signal at a different location;

at least one processor; and

at least one memory including computer program code

the at least one memory and the computer program code configured to, with the at least one processor, cause the apparatus at least to perform:

estimating an in-vivo distance, from a heart to a first one of a plurality of sensors in dependence upon a determined unconstrained body position of the subject, wherein the distance causes a time difference, dependent upon a pulse wave velocity, between detecting a heart-related signal and detecting a vascular signal at the first one of a plurality of distributed sensors.

According to various, but not necessarily all, embodiments of the invention there is provided examples as claimed in the appended claims.

BRIEF DESCRIPTION

For a better understanding of various examples that are useful for understanding the detailed description, reference will now be made by way of example only to the accompanying drawings in which:

Fig. 1 illustrates an example of a method for calculating a pulse wave velocity of a subject;

Fig. 2 illustrates an example of a system for calculating a pulse wave velocity of a subject;

Fig. 3 illustrates an example of a controller for calculating a pulse wave velocity of a subject;

Fig. 4 illustrates examples of sensors for performing the method;

Fig. 5 illustrates examples of the sensors for performing the method; and

Fig. 6 illustrates an apparatus comprising embedded sensors including at least the plurality of distributed embedded sensors used for detecting the vasculature signal.

DETAILED DESCRIPTION

In the examples described below, methods, apparatuses, computer programs and systems are described which may be used to measure a pulse wave velocity of a subject, for example a human subject. The pulse wave velocity is measured without a requirement to constrain the position of the subject relative to the sensors used or to constrain the position of the sensors used relative to the subject. The subject has an unconstrained body position relative to the sensors used. The subject may therefore be unaware that the measurement is in progress.

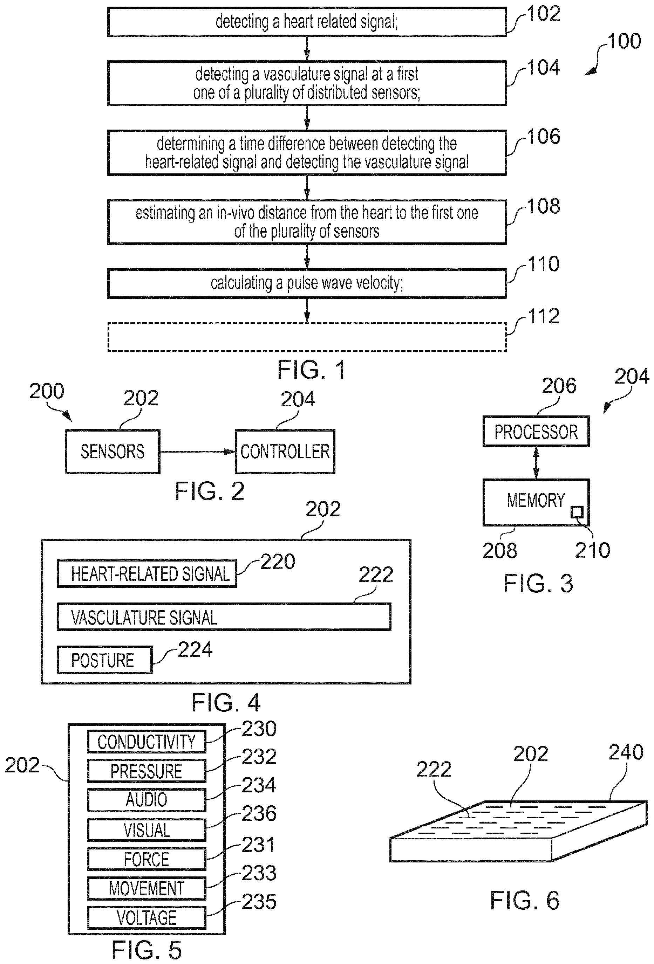

Fig. 1 illustrates an example of a method 100. The method 100 is for calculating a pulse wave velocity of a subject without constraining the body position of the subject relative to sensors used.

In some but not necessarily all examples, the sensors are not attached to the subject's body nor does the subject have to adopt a particular constrained body position relative to the sensors.

At block 102, the method 100 comprises detecting a heart-related signal of a subject.

At block 104, the method 100 comprises detecting a vasculature signal of the subject at a first one of a plurality of distributed sensors.

At block 106, the method 100 comprises determining a time difference between detecting the heart-related signal and detecting the vasculature signal.

At block 108, the method 100 comprises using a controller to automatically estimate an in-vivo distance from the heart of the subject to the first one of the plurality of sensors in dependence upon a determined unconstrained body position of the subject.

At block 110, the method 100 comprises calculating a pulse wave velocity by dividing the estimate of the in-vivo distance from the heart of the subject to the first one of the plurality of sensors by the time difference between detecting the heart-related signal and detecting the vasculature signal.

It should be appreciated that the first one of the plurality of distributed sensors that detects the vasculature signal of the subject may be any one of the plurality of distributed sensors. The identity of the first one of the plurality of distributed sensors will change with the changing body position of the subject.

The blocks 104, 108 may therefore alternatively be described as detecting a vasculature signal of the subject at any one of the plurality of distributed sensors and using a controller to automatically estimate an in-vivo distance from the heart of the subject to the sensor used to detect the vasculature signal, in dependence upon a determined unconstrained body position of the subject.

The heart-related signal of a subject may relate to any phase of the heart cycle. In some but not necessarily all examples it may be a signal produced by or as a consequence of ventricular contraction.

The vasculature signal of the subject may be a signal produced by an arterial pulse wave. In some but not necessarily all examples it may be a signal dependent upon a change in arterial volume.

Fig. 2 illustrates an example of a system 200 comprising sensors 202 and a controller 204. The system 200 is configured to perform the method 100 as described with reference to Fig. 1.

The sensors 202 comprise one or more sensors for detecting the heart-related signal, a plurality of distributed sensors for detecting a vasculature signal and sensors for determining a subject's body position (posture).

The plurality of sensors are spatially distributed over an area or volume such that a vasculature signal can be detected, at different ones of the plurality of sensors, as the subject changes posture.

A controller 204 is configured to receive sensing data from the sensors 202. In some examples, the controller 204 is configured to perform the whole of the method 100 including the detection of the heart-related signal (block 102) and the detection of the vasculature signal (block 104) from the data provided by the sensors 202. In other examples, the sensors 202 themselves may detect the heart-related signal and the vasculature signal and provide timing information to the controller 204 such that it can perform the block 106 of the method 100.

The controller 204 is configured to estimate an in-vivo distance D from a heart of the subject to a first one of a plurality of sensors 202. The distance causes a time difference T between detecting a heart-related signal and detecting a vasculature signal at the first one of a plurality of distributed sensors.

In some but not necessarily all examples, the in-vivo distance D is determined from the unconstrained posture of the subject. The distance D may be determined with body posture image recognition algorithms or by matching an entry in a database to detected posture sensor data. The database may comprise a plurality of entries each associating different reference posture sensor data p to different distances D(p). Querying the database with the detected posture sensor data p' returns the distance D(p'). In some but not necessarily all examples the database may return the distance D(p") where p" is the reference posture sensor data that is most similar to the detected posture sensor data p'. Similarity may be determined in different ways, for example, pattern matching, principal component analysis, neural networks, hidden Markov models or other approaches may be used.

In some but not necessarily all examples, the database may be stored remotely and offered as a centralized service to multiple users simultaneously. In other examples, the database may be stored locally at the controller 204.

In some but not necessarily all examples, the controller 204 is configured to calculate a pulse wave velocity using the determined time difference T and the estimated in-vivo distance D. If the heart-related signal relates to ventricular output then the distance D is divided by the time T. If the heart-related signal relates to a different phase of the cardiac cycle, the time T may be adjusted and the pulse wave velocity determined by dividing the distance D by the adjusted time T'.

In some examples, the controller 204 may perform all of the blocks 102, 104, 106, 108, 110 of Fig. 1. As described above, in other examples it may perform only blocks 106 to 110. It other examples, it may only perform one or more of blocks 106 to 110, forming part of a distributed system comprising multiple components that in combination perform the blocks 102 to 110. For example, one controller may perform block 106, and the same or a different controller may perform block 108 and the same or a different controller may perform block 110.

In some, but not necessarily all examples, the sensors 202 may, in addition, comprise electrocardiogram sensors for example capacitive electrocardiogram sensors. These may be used for accurately monitoring the cycle of a human heart and may be used to detect the heart-related signal during normal use or during a calibration phase.

In some, but not necessarily all examples, the sensors 202 may, in addition, comprise sensors measuring the mechanical ventricular contraction, for example, Ballistocardiogram (BCG) or seism cardiogram (SCG) sensors.

Referring back to Fig. 1, after block 110, the method 100 may, in some but not necessarily all examples, comprise a further block or blocks 112 that use the calculated pulse wave velocity.

For example, at block 112, the method 100 may comprise a control step such as controlling the storage of the pulse wave velocity as a continuous record over time and/or sending the calculated pulse wave velocity to a remote location for storage or processing and/or performing a further calculation in relation to the pulse wave velocity and/or in relation to the calculated pulse wave velocity or in relation to the further calculation based upon the pulse wave velocity, generating a warning or an alert.

It may, for example, be possible to use the pulse wave velocity to calculate an indicator of arterial stiffness. A theoretical model based on the Moens-Korteweg equation relates the ratio of the square of the pulse wave velocity v to the Young's modulus E of arterial stiffness to a constant c: $v^2 = c.E$.

It is therefore possible to obtain and store an indicator of arterial stiffness for a subject and also to monitor and store changing arterial stiffness over time for a subject.

It is also possible to convert the calculated pulse wave velocity to a systolic blood pressure estimate. This conversion may be based upon a theoretical model which, in turn, is based upon the Moens-Korteweg equation.

$$P = a \log_e \left\{ b / \left[ (d/v - c)^2 - 1 \right] \right\}$$

This equation relates the systolic blood pressure P to the calculated pulse wave velocity v using the empirically-determined constants a, b, c, d. The constants a, b, c, d may, for example, be determined using a calibration process in which both systolic blood pressure and the pulse wave velocity are measured. The systolic blood pressure may, for example be measured using a standard occlusion method and the pulse wave velocity may be measured separately as described above.

It is therefore possible to obtain and store an indicator of systolic blood pressure for a subject and also to monitor and store changing systolic blood pressure over time for a subject.

Implementation of the controller 204 may be as controller circuitry. The controller 204 may be implemented in hardware alone, have certain aspects in software including firmware alone or can be a combination of hardware and software (including firmware).

As illustrated in Fig. 3 the controller 204 may be implemented using instructions that enable hardware functionality, for example, by using executable instructions of a computer program 210 in a general-purpose or special-purpose processor 206 that may be stored on a computer readable storage medium (disk, memory etc) to be executed by such a processor 206.

The processor 206 is configured to read from and write to the memory 208. The processor 206 may also comprise an output interface via which data and/or commands are output by the processor 206 and an input interface via which data and/or commands are input to the processor 206.

The memory 208 stores a computer program 210 comprising computer program instructions (computer program code) that controls the operation of the controller 204 when loaded into the processor 206. The computer program instructions, of the computer program 210, provide the logic and routines that enables the apparatus to perform the methods illustrated in Fig. 1. The processor 206 by reading the memory 208 is able to load and execute the computer program 210.

The apparatus 204 therefore comprises:

at least one processor 206; and
at least one memory 208 including computer program code
the at least one memory 208 and the computer program code configured to, with the at least one processor 206, cause the apparatus 204 at least to perform:

estimating an in-vivo distance, from a heart to a first one of a plurality of sensors in dependence upon a determined unconstrained body position of the subject, wherein the distance causes a time difference, dependent upon a pulse wave velocity, between detecting a heart-related signal and detecting a vascular signal at the first one of a plurality of distributed sensors.

The computer program 210 may arrive at the controller 204 via any suitable delivery mechanism. The delivery mechanism may be, for example, a non-transitory computer-readable storage medium, a computer program product, a memory device, a record medium such as a compact disc read-only memory (CD-ROM) or digital versatile disc (DVD), an article of manufacture that tangibly embodies the computer program 210. The delivery mechanism may be a signal configured to reliably transfer the computer program 210. The controller may propagate or transmit the computer program 210 as a computer data signal.

Although the memory 208 is illustrated as a single component/circuitry it may be implemented as one or more separate components/circuitry some or all of which may be integrated/removable and/or may provide permanent/semi-permanent/ dynamic/cached storage.

Although the processor 206 is illustrated as a single component/circuitry it may be implemented as one or more separate components/circuitry some or all of which may be integrated/removable. The processor 206 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' refers to all of the following:

(a) hardware-only circuit implementations (such as implementations in only analog and/or digital circuitry) and
(b) to combinations of circuits and software (and/or firmware), such as (as applicable): (i) to a combination of processor(s) or (ii) to portions of processor(s)/software (including digital signal processor(s)), software, and memory(ices) that work together to cause an apparatus, such as a mobile phone or server, to perform various functions and
(c) to circuits, such as a microprocessor(s) or a portion of a microprocessor(s), that require software or firmware for operation, even if the software or firmware is not physically present.

This definition of 'circuitry' applies to all uses of this term in this application, including in any claims. As a further example, as used in this application, the term "circuitry" would also cover an implementation of merely a processor (or multiple processors) or portion of a processor and its (or their) accompanying software and/or firmware. The term "circuitry" would also cover, for example and if applicable to the particular claim element, a baseband integrated circuit or applications processor integrated circuit for a mobile phone or a similar integrated circuit in a server, a cellular network device, or other network device.

Fig. 4 illustrates examples of sensors 202. In this example, the sensors 202 comprise:

i) one or more sensors 220 configured to sense the heart-related signal,
ii) the plurality of distributed sensors 222 configured to detect a vasculature signal at different positions for different postures of the subject and
iii) sensors 224 for detecting the posture of the subject.

The sensors 220, 222, 224 may, in some examples, be independent, different sensors and in some examples one or more of the sensors may be the same sensor that performs two or more functions.

The sensors 220, 222, 224 may, in some examples, be unconstrained sensors that do not have to be in a particular spatial relationship to the subject to perform the function described. Thus the heart-related signal, the vasculature signal and the posture may be determined for different arbitrary postures of the subject.

The one or more sensors 220 for sensing the heart-related signal may, for example, comprise movement sensors, force sensors, electrical voltage sensors, impedance (or conductivity) sensors, pressure sensors and/or audio sensors.

A movement sensor may be used to detect the movement of the subject's heart. Such a sensor may be described as a ballistocardiography sensor as it detects the body movement arising from the blood flow in arteries or as a seismocardiography sensor as it detects the chest wall movement arising from the heart movement. Such sensors may be implemented using, for example, an accelerometer sensor or a gyroscope sensor.

A force or pressure sensor may be used to detect a ventricular contraction of the subject's heart. Such a sensor may be described as a ballistocardiography sensor as it detects the force or pressure arising from body movement or as seismocardiography sensor as it detects the force or pressure arising from chest wall movement arising from the heart movement. Such sensors may be implemented using, for example, a piezoelectric sensor, an electromechanical film sensor, a strain gauge sensor, a piezoresistive sensor, a capacitive sensor or a fluid pressure sensor.

An electrical voltage sensor may be used to detect the electrical signal generated by cardiac cycle. Such a sensor may be described as an electrocardiography sensor as it detects the electrical signal generated by the subject's heart muscles. Such sensors may be implemented using, for example, resistive and/or capacitive coupling to the user's body.

An impedance (or conductivity) sensor may be used to detect the impedance change generated by cardiac cycle. Such a sensor may be described as an impedancecardiography sensor as it detects the impedance change generated by increased fluid i.e. blood volume in the measurement area. Such sensors may be implemented using, for example, resistive and/or capacitive coupling to the user's body.

An audio sensor, for example a microphone, may be configured to detect audio arising from the heart cycle of the subject. Such a sensor may be described as a phonocardiography sensor.

The plurality of distributed sensors 222 are configured to sense a vasculature signal, for example an arterial pulse, of the subject at different locations such that as the posture of the subject changes it remains possible to sense a vasculature signal at one of the plurality of distributed sensors 222. The plurality of distributed sensors 222 may for example be contact sensors configured for impedance plethysmography, force sensors, pressure sensors and/or audio sensors.

The posture sensors 224 are configured to determine a posture of the subject. The posture of the subject may change because the body position of the subject is unconstrained for determination of the pulse wave velocity. The determination of the subject's posture may, for example, be determined by using spatially distributed pressure sensors, spatially distributed force sensors, spatially distributed impedance sensors, spatially distributed capacitance sensors, spatially distributed inductance sensors or by using a visual sensor and computer vision analysis or reflected structured light analysis to determine the position of the subject's limbs. The visual sensor may operate in the visible spectrum or the infrared spectrum for example.

Fig. 5 illustrates examples of the sensors 202, described from a different perspective to that described in relation to Fig. 4. In this example, the sensors 202 may comprise one or more of the following sensors:

i) conductivity sensors 230 for sensing a vasculature signal, for example a pulse. These conductivity sensors may, for example, be configured for impedance plethysmography;
ii) pressure sensors 232 configured to detect the heart-related signal and/or the vasculature signal (the pulse) and/or the subject's posture;
iii) force sensors 231 configured to detect the heart-related signal and/or the vasculature signal (the pulse) and/or the subject's posture;
iv) movement sensors 233 configured to detect the heart-related signal and/or the vasculature signal (the pulse);
v) electrical voltage sensors 235 configured to detect the electrical signal generated by cardiac cycle;
vi) audio sensors 234 configured to detect the heart-related signal and/or the vasculature signal (the pulse);
vii) visual sensors 236 for example a camera operating in the visible spectrum or the infrared spectrum, configured to detect a subject's posture.

One or more of the different sensors described may be used to detect the heart-related signal, the vasculature signal at different positions and the subject's posture.

Pressure, force and/or conductivity sensors may be re-used for determining the subject's posture and for determining one or more circulatory signals (the heart-related signal and/or the vasculature signal).

Fig. 6 illustrates an apparatus 240 comprising sensors 202 including at least the plurality of distributed sensors 222 used for detecting the vasculature signal. It may, in addition, comprise sensors 220 for detecting the heart-related signal and sensors 224 for detecting the posture of the subject.

The apparatus 240 is configured such that the sensors 202 are proximal to the subject when the subject is using the apparatus 240. The apparatus 240 may, for example, be furniture, furnishings or clothes that are proximal to the subject. As such, it is not therefore necessary to attach sensors 202, 222 to the subject and there is the possibility of relative movement between the sensors 202, 222 and the subject. There is no requirement to constrain movement of the sensors 202, 222 relative to the subject nor to constrain movement of the subject relative to the sensors 202, 222.

In the illustrated example, the apparatus 240 is a bed or a mattress in which the sensors 202, 222 are embedded. In alternative examples the apparatus 240 may be a sheet for a mattress in which the sensors 202, 222 are embedded.

It is possible to embed the sensors 202, 222 within a sheet or a mattress by using conductive stripes and/or electrical yarn. It is for example possible to create the plurality of distributed sensors 222 as electrical contact sensors suitable for impedance plethysmography.

The blocks illustrated in Fig. 1 may represent steps in a method and/or sections of code in the computer program 210. The illustration of a particular order to the blocks does not necessarily imply that there is a required or preferred order for the blocks and the order and arrangement of the block may be varied. Furthermore, it may be possible for some blocks to be omitted.

Where a structural feature has been described, it may be replaced by means for performing one or more of the functions of the structural feature whether that function or those functions are explicitly or implicitly described.

The recording of data may comprise only temporary recording, or it may comprise permanent recording or it may comprise both temporary recording and permanent recording. Temporary recording implies the recording of data temporarily. This may, for example, occur during sensing, occur at a dynamic memory, occur at a buffer such as a circular buffer, a register, a cache or similar. Permanent recording implies that the data is in the form of an addressable data structure that is retrievable from an addressable memory space and can therefore be stored and retrieved until deleted or over-written, although long-term storage may or may not occur. The use of the term 'store', 'storage' etc. in relation to data relates to permanent recording of the data.

As used here 'module' refers to a unit or apparatus that excludes certain parts/components that would be added by an end manufacturer or a user. The controller 204 may be a module.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one.." or by using "consisting".

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. A method comprising:

   determining a time difference between detecting a heart-related signal of a subject and detecting a vasculature signal of the subject at a first one of a plurality of distributed sensors;
   using a controller to automatically estimate an in-vivo distance from the heart of the subject to the first one of the plurality of sensors in dependence upon a determined unconstrained body position of the subject; and
   calculating a pulse wave velocity.

2. A method as claimed in claim 1, wherein the plurality of sensors comprises contact sensors for sensing a pulse using impedance plethysmography.

3. A method as claimed in claim 1 or 2, wherein the plurality of sensors are within a bed or are embedded in a sheet or a mattress for a bed.

4. A method as claimed in claim 1, 2 or 3, wherein the plurality of sensors is formed from a plurality of conductive stripes.

5. A method as claimed in any preceding claim, wherein the plurality of sensors comprises electrical yarn.

6. A method as claimed in any preceding claim, wherein the heart-related signal is for ventricular contraction.

7. A method as claimed in any preceding claim, wherein the heart-related signal is detected using one or more of: movement sensors for measuring the movement arising from a cycle of the heart or using, force sensors for measuring force arising from ventricular contraction of the heart, pressure sensors for measuring pressure arising from ventricular contraction of the heart, and audio sensors for detecting audio arising from a cycle of the heart.

8. A method as claimed in any preceding claim, wherein sensors are used for determining the subject's body position and estimating the in-vivo distance from the heart of the subject to the first one of the plurality of sensors.

9. A method as claimed in claim 8, wherein the sensors for determining a subject's body position are conductivity sensors, force sensors and/or pressure sensors used to detect the heart-related signal.

10. A method as claimed in any preceding claim further comprising converting the pulse wave velocity to an estimate of arterial stiffness.

11. A method as claimed in any preceding claim further comprising converting the calculated pulse wave velocity to an estimate of systolic blood pressure.

12. A method as claimed in any preceding claim further comprising using electrocardiograph sensors to detect a cycle of the heart.

13. An apparatus comprising means for enabling the performance of any of the methods claimed in claims 1 to 12.

14. A computer program that when run by a processor enables the processor to estimate an in-vivo distance, from a heart to a first one of a plurality of sensors in dependence upon a determined unconstrained body position of the subject, wherein the distance causes a time difference, dependent upon a pulse wave velocity, between detecting a heart-related signal and detecting a vascular signal at the first one of a plurality of distributed sensors.

15. A system comprising:

    means for detecting a heart-related signal;
    means for detecting a vasculature signal at a first one of a plurality of distributed sensors;
    means for determining a time difference between detecting the heart-related signal and detecting the vasculature signal;
    means for automatically estimating an in-vivo distance from the heart of the subject to the first one of the plurality of sensors in dependence upon a determined unconstrained body position of the subject; and
    means for calculating a pulse wave velocity using the determined time difference and the estimated in-vivo distance.

detecting a heart related signal; ⟋102

detecting a vasculature signal at a first
one of a plurality of distributed sensors; ⟋104

⟋100

determining a time difference between detecting the
heart-related signal and detecting the vasculature signal ⟋106

estimating an in-vivo distance from the heart to the first one
of the plurality of sensors ⟋108

calculating a pulse wave velocity; ⟋110

⟋112

FIG. 1

200   202   204

| SENSORS | → | CONTROLLER |

FIG. 2

206   204

PROCESSOR

MEMORY □

208   210

FIG. 3

202

HEART-RELATED SIGNAL ⟋220   222

VASCULATURE SIGNAL

POSTURE ⟋224

FIG. 4

202

| CONDUCTIVITY | ⟋230 |
| PRESSURE | ⟋232 |
| AUDIO | ⟋234 |
| VISUAL | ⟋236 |
| FORCE | ⟋231 |
| MOVEMENT | ⟋233 |
| VOLTAGE | ⟋235 |

FIG. 5

222   202   240

FIG. 6

Europäisches
Patentamt

European
Patent Office

Office européen
des brevets

**EUROPEAN SEARCH REPORT**

Application Number

EP 17 17 9202

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/078735 A1 (KONINKL PHILIPS NV [NL]) 4 June 2015 (2015-06-04) | 1-8, 10-15 | INV. A61B5/021 A61B5/02 |
| Y | * page 9, line 7 - page 12, line 18 * <br> * page 14, line 21 - page 15, line 11 * <br> * page 16, line 11 - page 17, line 11 * <br> * page 18, lines 9-20 * <br> * figures * | 9 | ADD. A61B5/00 A61B5/024 A61B5/0285 A61B5/0295 A61B5/11 |
| | ----- | | |
| Y | US 2011/112442 A1 (MEGER GUY [IL] ET AL) 12 May 2011 (2011-05-12) <br> * paragraphs [0095], [0206], [0259] * <br> * figure 1 * | 9 | |
| | ----- | | |
| X | US 2016/089081 A1 (MORRIS DAN [US] ET AL) 31 March 2016 (2016-03-31) <br> * paragraphs [0023] - [0028] * <br> * paragraphs [0040] - [0045] * | 1-8,10, 12-15 | |
| | ----- | | |
| A | US 2016/174852 A1 (HE DAVID DA [US] ET AL) 23 June 2016 (2016-06-23) <br> * paragraphs [0080], [0117] - [0126] * <br> * figure 10 * | 1-15 | TECHNICAL FIELDS SEARCHED (IPC) |
| | ----- | | A61B |
| A | WO 03/082111 A1 (OCEANIT LAB INC [US]) 9 October 2003 (2003-10-09) <br> * page 26, line 21 - page 27, line 3 * <br> * page 28, lines 10-11 * <br> * figure 14 * | 1-15 | |
| | ----- | | |

The present search report has been drawn up for all claims

| Place of search | Date of completion of the search | Examiner |
|---|---|---|
| The Hague | 8 December 2017 | Bataille, Frédéric |

## ANNEX TO THE EUROPEAN SEARCH REPORT
## ON EUROPEAN PATENT APPLICATION NO.

EP 17 17 9202

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2017

| Patent document cited in search report | | | Publication date | Patent family member(s) | | Publication date |
|---|---|---|---|---|---|---|
| WO 2015078735 | A1 | | 04-06-2015 | CA 2931377 A1 | | 04-06-2015 |
| | | | | CN 105792742 A | | 20-07-2016 |
| | | | | EP 3073905 A1 | | 05-10-2016 |
| | | | | JP 2016539697 A | | 22-12-2016 |
| | | | | US 2017164904 A1 | | 15-06-2017 |
| | | | | WO 2015078735 A1 | | 04-06-2015 |
| US 2011112442 | A1 | | 12-05-2011 | CN 102113034 A | | 29-06-2011 |
| | | | | EP 2286395 A2 | | 23-02-2011 |
| | | | | EP 2701131 A2 | | 26-02-2014 |
| | | | | JP 2012502671 A | | 02-02-2012 |
| | | | | US 2011112442 A1 | | 12-05-2011 |
| | | | | WO 2009138976 A2 | | 19-11-2009 |
| US 2016089081 | A1 | | 31-03-2016 | CN 106714674 A | | 24-05-2017 |
| | | | | EP 3200684 A1 | | 09-08-2017 |
| | | | | US 2016089081 A1 | | 31-03-2016 |
| | | | | WO 2016053751 A1 | | 07-04-2016 |
| US 2016174852 | A1 | | 23-06-2016 | NONE | | |
| WO 03082111 | A1 | | 09-10-2003 | AU 2002311781 A1 | | 13-10-2003 |
| | | | | CA 2479972 A1 | | 09-10-2003 |
| | | | | CN 1625368 A | | 08-06-2005 |
| | | | | EP 1494586 A1 | | 12-01-2005 |
| | | | | HK 1075190 A1 | | 09-04-2010 |
| | | | | JP 4344247 B2 | | 14-10-2009 |
| | | | | JP 2006504443 A | | 09-02-2006 |
| | | | | WO 03082111 A1 | | 09-10-2003 |

EPO FORM P0459

For more details about this annex : see Official Journal of the European Patent Office, No. 12/82